# EUROPEAN PATENT APPLICATION

(11) **EP 1 500 933 A1**
(43) Date of publication of application: **26.01.2005**
(21) Application number: 03717688.0
(22) Date of filing: 22.04.2003
(51) Int. Cl.: G01N 33/53, C12M 1/00

(54) **DEVICE, METHOD, AND KIT FOR GENE DETECTION**

(30) Priority: 22.04.2002 JP 2002119560
(71) Applicant: Hokuto Scientific Industry, Co., Ltd., Toyama-shi, Toyama 930-0897 (JP); Tamiya, Eiichi, Nomi-gun, Ishikawa 923-1211 (JP)
(72) Inventor: Tamiya, Eiichi, Tamiya Laboratory, Nomi-gun, Ishikawa 923-0897 (JP); Kobayashi, Masaaki, Tamiya Laboratory, Nomi-gun, Ishikawa 923-0897 (JP); NAGASHIO, Ichiro, Toyama-shi, Toyama 930-0897 (JP)
(74) Representative: Patentanwälte Ruff, Wilhelm, Beier, Dauster & Partner
(86) International application number: PCT/JP2003/005085
(87) International publication number: WO 2003/089929

(57) **Abstract**

A gene detection tool comprising a sealable reaction chamber and a sealable detection chamber and a partition member positioned between the reaction chamber and the detection chamber that separates the reaction chamber and the detection chamber. The partition member is capable of unsealing; the reaction chamber is comprised of at least two members capable of forming a sealed reaction chamber by mutually fitting an opening means provided therein; and the detection chamber or reaction chamber comprises a gene detection element. A gene detection method in which a gene amplification operation and a gene detection operation are conducted in a sealed state using a gene detection device comprising a sealable reaction chamber and a sealable detection chamber, having a partition member which is positioned between the reaction chamber and the detection chamber and which separates the reaction chamber from the detection chamber, with test sample and gene amplification reaction solution being packed in said reaction chamber and a gene-binding agent capable of binding with a gene and generating a detectable signal being packed in said detection chamber. (1) At least the reaction chamber is imparted with conditions capable of amplifying the gene to be detected; (2) following the operation of (1), the partition member is unsealed while maintaining the gene detection tool in a sealed state; (3) through a passage created in the partition member by the unsealing, the solution in the reaction chamber is brought into contact with the gene-binding agent capable of binding the gene in the detection chamber and generating a detectable signal; and (4) the signal obtained is detected. A gene detection kit comprising the gene detection tool, a gene amplification reaction solution or gene amplification reagent, and a gene-binding agent capable of binding a gene and generating a detectable signal.

## Description

### Technical Field

The present invention relates to a gene detection tool, detection method, and detection kit. The gene detection tool and detection method of the present invention permit the detection of genes in a closed system from trace test samples. Thus, they enhance safety in the course of handling samples derived from patients at medical treatment facilities and clinical examination facilities.

### Background Art

The technique of hybridization, particularly southern hybridization (the southern blotting method) has generally been employed to detect DNA with a specific DNA sequence.

In the southern hybridization method, the DNA in a test sample is fragmented with a restriction enzyme, the DNA fragments are separated by electrophoresis in agarose gel or polyacrylamide gel based on size, the individual DNA fragments separated on the gel are denatured into single-stranded DNA, and these strands are immobilized on a nylon filter, nitrocellulose paper, or the like. Next, the single strands of denatured DNA are hybridized with labeled probe DNA (for example, radioactive isomer elements, fluorescent materials, or light-emitting substances are employed as labels). Subsequently, the filter is washed to remove labeled probe DNA that has not hybridized and signals from the labels of the double-stranded DNA formed by hybridization are detected, permitting the detection of DNA having a specific DNA sequence.

However, in the above hybridization method, not only is a relatively large quantity of test samples required for electrophoresis, but each of the operations is conducted in an open system. Accordingly, for example, when handling a sample (for example, a blood sample) from a patient at a medical treatment facility or clinical examination facility, there is a possibility of the processor being infected when the sample has been contaminated by a dangerous pathogenic bacterium or virus. Difficult issues are also presented by the processing of waste material (gel, rinse solutions, and the like) generated by examination.

Electrochemical detection methods employing sensors with electrodes and intercalating agents are another known technique of detecting DNA having a specific DNA sequence.
For example, Japanese Unexamined Patent Publication (KOKAI) Heisei No. 9-288080 describes a method of reacting probe DNA immobilized onto a surface of an electrode with sample DNA in the presence of an intercalating agent and measuring the current of the electrode after the reaction to detect the presence of hybrid DNA formed by the probe DNA and sample DNA.

Japanese Unexamined Patent Publication (KOKAI) Heisei No. 5-285000 discloses a gene detection method of immersing an electrode in a sample solution containing sample DNA in a denatured single-stranded state to cause the sample DNA to adsorb onto the surface of the electrode, immersing the electrode in a reaction cell in which probe DNA has been collected, controlling the temperature of the probe solution to conduct hybridization, immersing the electrode in a detection cell in which a solution containing an intercalating agent has been maintained, and detecting the electrochemical signal originating from the bound intercalating agent that has recognized the double-stranded DNA formed on the surface of the electrode. This publication further discloses that when sample DNA is present in trace quantity, the DNA can be amplified by PCR prior to detection.

Accordingly, the above-cited electrochemical detection methods have the same drawbacks as described above when samples derived from patients are handled by medical treatment facilities and clinical examination facilities because the operations are conducted in open systems.

Closed system examination tools are known for use with special samples such as feces. For example, Japanese Unexamined Patent Publication (KOKAI) Heisei No. 8-54390 describes a device for detecting occult blood in stool comprising a closed solution chamber for preparing a stool suspension and a closed sample chamber for detecting antigen-antibody complex. When employing this detection device, the solution chamber and sample chamber are closed off from each other up through preparation of the stool suspension, but following preparation of the stool suspension, a film capable to rupturing is ruptured and the solution chamber and sample chamber are processed within a single sealed chamber.
Japanese Unexamined Patent Publication (KOKAI) No. 2000-146957 describes a smear examination tool primarily for bacteria detection.

However, no closed-system examination tool for the detection of genetic material such as DNA is known. The closed-system examination tools described in the above-cited publications all assume a relatively large quantity of sample, and are not techniques of detection in which the sample (for example, bacteria) is increased in a sample preparation chamber or the like provided in the detection tool prior to detection. Further, the means of rupturing the film capable of rupturing is complicated and manufacturing costs are high.

Accordingly, the object of the present invention is to provide a gene detection technique, that is, a gene detection tool, gene detection method, and detection kit, permitting the detection of genes having a specific DNA sequence from a trace amount of sample, reducing the risk of contagion of processors when handling dangerous samples in medical treatment facilities, clinical examination facilities, or the like, facilitating the handling of waste material generated by examination, and permitting size reduction through structure simplification.

### Disclosure of the Invention

The above-stated object is achieved by the present invention as follows.

The present invention relates to a gene detection tool comprising a sealable reaction chamber and a sealable detection chamber and a partition member (wall) positioned between the reaction chamber and the detection chamber that separates the reaction chamber and the detection chamber, wherein
the partition member is capable of unsealing;
the reaction chamber is comprised of at least two members capable of forming a sealed reaction chamber by mutually fitting an opening means provided therein; and
the detection chamber or reaction chamber comprises a gene detection element.

In the gene detection tool of the present invention, the partition member comprises a groove-shaped notch in a portion of the surface thereof. At least the reaction chamber and the detection chamber adjacent to the partition member are formed of a flexible material. Thus, the operation of applying pressure to the tool from the exterior ruptures at least a portion of the notch, unsealing the partition member.

In the gene detection tool of the present invention, the reaction chamber is comprised of a cylindrical member one end of which communicates with the detection chamber and is sealed off by the partition member and the other end of which is comprised of an opening means, and a cylindrical member having a bottom, one end of which is closed and the other end of which is an opening means. These two opening means fit together, making it possible to seal off the reaction chamber. Further, the reaction chamber may also have a gene detection member on the end that is closed off by the cylindrical member having a bottom.

In the gene detection tool of the present invention, one end of the detection chamber communicates with the reaction chamber and is sealed off by the partition member. The other end thereof is comprised of an opening means in the form of a cylindrical member, it being possible for a tightly fitting cover comprising a gene detection member to be provided in the opening means.

In the gene detection tool of the present invention, the reaction chamber can be packed with test sample and gene amplification reaction solution.

In the gene detection tool of the present invention, the detection chamber can be packed with a gene-binding agent that binds genes and generates a detectable signal. Further, a passage for mixing the test sample and gene amplification reaction solution in the reaction chamber with the gene-binding agent that binds genes and generates a detectable signal in the detection chamber can be created by unsealing the partition member.

In the gene detection tool of the present invention, the gene detection member (chamber) can be an electrochemical detection member or a fluorescence-detecting member.

More specifically, the gene detection tool of the present invention comprises a mutually independent sealable reaction chamber and detection chamber, and a partition member positioned between the reaction chamber and the detection chamber that seals off the reaction chamber and detection chamber from each other, and is characterized in that:
(a) the reaction chamber comprises an opening means permitting the insertion of a test sample potentially containing a gene to be detected and a gene amplification reaction solution;
(b) the detection chamber comprises an opening means permitting the insertion of a gene-binding agent that binds a gene and generates a detectable signal;
(c) while maintaining the overall sealed state of the gene detection tool comprising the reaction chamber and detection chamber, the partition member can be unsealed by an operation from the exterior; and
(d) when the partition member is unsealed, the passage generated by this unsealing allows the solution in the reaction chamber to move into the detection chamber.

In the gene detection tool of the present invention, the gene-binding agent may be selected from among the group consisting of:
(a) electrochemically active agents capable of specifically binding genes;
(b) fluorescent dyefluorescent dyes capable of specifically binding genes; and
(c) probes having a region capable of sequence-specifically binding a gene to be detected and pairs of fluorescent groups and quenching groups, incapable of generating fluorescence prior to binding the gene to be detected but capable of generating fluorescence once bound to the gene to be detected.

The present invention further relates to a gene detection method in which a gene amplification operation and a gene detection operation are conducted in a sealed state using a gene detection device comprising a sealable reaction chamber and a sealable detection chamber, having a partition member positioned between the reaction chamber and the detection chamber separating the reaction chamber from the detection chamber, with test sample and gene amplification reaction solution being packed in said reaction chamber and a gene-binding agent capable of binding with a gene and generating a detectable signal being packed in said detection chamber, wherein:
(1) at least the reaction chamber is imparted with conditions capable of amplifying the gene to be detected;
(2) following the operation of (1), the partition member is unsealed while maintaining the gene detection tool in a sealed state;
(3) through a passage created in the partition member by the unsealing, the solution in the reaction chamber is brought into contact with the gene-binding agent capable of binding the gene in the detection chamber and generating a detectable signal; and
(4) the signal obtained is detected.

In the above gene detection method, the gene-binding agent may be selected from among the group consisting of:
(a) electrochemically active agents capable of specifically binding genes;
(b) fluorescent dyefluorescent dyes capable of specifically binding genes; and
(c) probes having a region capable of sequence-specifically binding a gene to be detected and pairs of fluorescent groups and quenching groups, incapable of generating fluorescence prior to binding the gene to be detected but capable of generating fluorescence once bound to the gene to be detected.

In the above gene detection method, the signal may be detected by electrochemical detection or fluorescent detection.

In the above gene detection method, the gene amplification reaction solution can be prepacked into the reaction chamber and the test sample can be inserted into the reaction chamber prior to the operation of (1).

In the above gene detection method, the reaction chamber may have an opening means, the test sample and/or gene amplification reaction solution may be inserted into the reaction chamber through the opening means, and following insertion, the opening means can be closed to seal the reaction chamber.

In the above gene detection method, the gene-binding agent that binds a gene and generates a detectable signal can be packed in the detection chamber.

More specifically, the above gene detection method can be a method
characterized by comprising the steps of:
(1) inserting a test sample potentially containing the gene to be detected and the gene amplification reaction solution through an opening means into the reaction chamber and then closing the opening means to seal the reaction chamber;
(2) imparting conditions permitting amplification of the gene to be detected to the interior of the reaction chamber;
(3) following the elapse of a period of time adequate for amplification of the gene to be detected and while maintaining the gene detection tool comprising the reaction chamber and detection chamber in an overall sealed state, unsealing by an external operation the partition member that is positioned between the reaction chamber and the detection chamber and seals off the reaction chamber from the detection chamber;
(4) causing the reaction solution in the reaction chamber to move into the detection chamber through a passage created by this unsealing;
(5) contacting the gene-binding agent that binds a gene and generates a detectable signal with the reaction solution in the detection chamber; and
(6) detecting the signal obtained.

The present invention further relates to a gene detection kit comprising the gene detection tool of the present invention, a gene amplification reaction solution or gene amplification reagent, and a gene-binding agent capable of binding a gene and generating a detectable signal. The gene detection kit may comprise a means of purifying genes.

### Brief Description of the Drawings

Fig. 1 is a schematic section view of the gene detection tool of the present invention.
Fig. 2 is a schematic deal drawing of the gene detection tool of Fig. 1.
Fig. 3 is a plane view of the partition member contained in the gene detection tool of Fig. 1.
Fig. 4 is a sectional view along section line A-A of Fig. 3.
Fig. 5 is a schematic perspective view of the sealed state of the partition member contained in the gene detection tool of the present invention.
Fig. 6 is a schematic sectional view of the gene detection tool of the present invention after unsealing the partition member.
Fig. 7 is a schematic sectional view of the partition member contained in the gene detection tool of the present invention in another state prior to unsealing.
Fig. 8 is a schematic sectional view following unsealing of the partition member of Fig. 7.
Fig. 9 is a graph showing the detection results obtained by linear sweep voltammetry when hepatitis B virus was employed.

The numerals employed in the above drawings denote the following: 1: Reaction chamber; 1a: open end of reaction chamber; 2: electrochemical detection chamber; 2a: open end of detection chamber; 3: partition member; 4: fitting member; 5: cover member; 5a, 5b: ends of cover member; 6: gene amplification reaction solution; 7: solution containing gene-binding agent; 8: finger; 9: mixed solution; 10: gene detection tool; 11: cylinder with bottom; 11a, 11b: ends of cylinder with bottom; 12: cylinder with sections; 12a, 12b: ends of cylinder with sections; 31: thin film member; 31a: perimeter portion of thin film member; 32: groove; 33: center portion of thin film member; 34: closing film support cap; 34a: closing film; 34b: closing film support frame; 35: protrusion support cap; 35a: protrusion; 35b: protrusion support frame; 51: electrode; 52: stopper; 53: terminal.

### Best Mode of Implementing the Invention

Specific modes of the gene detection tool of the present invention are described below based on the appended drawings based on the use of an electrochemically active agent capable of specifically binding genes as the gene-binding agent. Direct application is possible when employing a fluorescent dye or probe capable of generating fluorescence as the gene-binding agent in modes other than the specific mode based on the use of electrochemically active agents in the description given below.

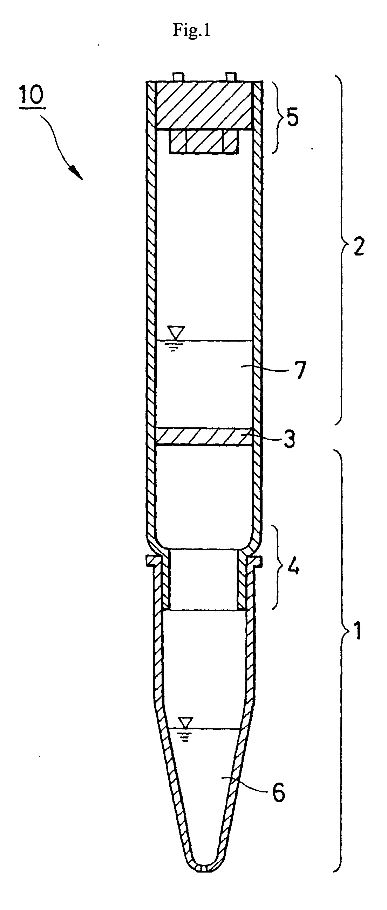

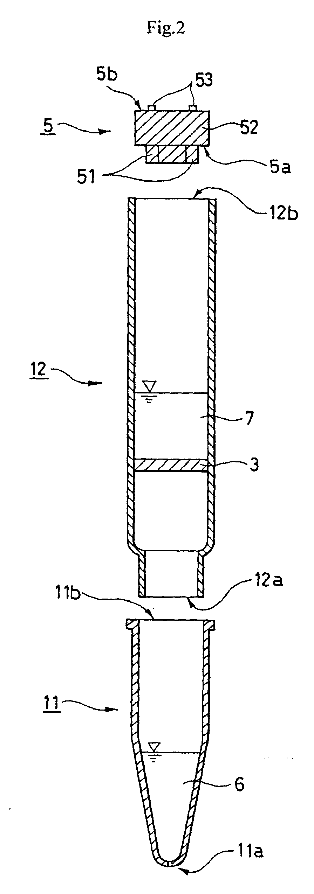

Fig. 1 is a schematic sectional view of the gene detection tool 10 of the present invention. Fig. 2 is a deal view of the same. As shown in Fig. 1, gene detection tool 10 comprises a reaction chamber 1, a detection chamber 2, and a partition member 3 positioned between reaction chamber 1 and detection chamber 2. That is, reaction chamber 1 and detection chamber 2 are separated by partition member 3. Reaction chamber 1 can have an opening means for reaction chamber 1 in the form of a fitting member 4 readily attachable and detachable by means of screws or the like, as well as reaction chamber I can contain a gene amplification reaction solution 6. When employing an electrochemically active agent as the gene-binding agent, detection chamber 2 is an electrochemical detection chamber, equipped at one end with a cover member 5 having an electrode 51 and containing in its interior a solution 7 containing a gene-binding agent. As shown in Fig. 1, partition member 3 seals off reaction chamber 1 from detection chamber 2 until being unsealed, described further below.

Gene detection tool 10 of the present invention shown in Fig. 1 may be assembled as shown in Fig. 2 out of a cylinder 11 with a bottom, a cylinder 12 having sections, and a cover member 5. Cylinder with bottom 11 is a cylinder that is closed at one end 11a and open at the other end 11b. Cylinder with sections 12 is a cylinder that is open at both ends 12a and 12b and has a partition member 3 similar to that found in bamboo sections at its center. The open end 11b of cylinder with bottom 11 and one end 12a of cylinder with sections 12 can be fitted together with screws or the like, forming a sealed reaction chamber 1. At the other end 11b of cylinder with sections 12, a cover member 5 can be mounted in a readily detachable manner with screws or the like. By fitting cover member 5 in place, a sealed detection chamber 2 can be formed.

When an electrochemically active agent is employed as the gene-binding agent, cover member 5 is equipped with an exposed electrode 51 on one end 5a of stopper 52 and equipped with an exposed terminal 53 on the other end 5b. Cover member 5 can be fitted in a readily detachable manner on one end 12b of cylinder with sections 12 by means of screws or the like positioned on a lateral surface of stopper 52. In the course of mounting cover member 5 on end 12b of cylinder with sections 12, it is fitted so that the end 5a on which electrode 51 is provided is contained within cylinder with sections 12 and the end 5b equipped with terminal 53 is positioned outside cylinder with sections 12.

Cover member 5 can be mounted on end 12b of cylinder with sections 12 to form a sealed detection chamber (electrochemical detection chamber) 2, and can be removed to provide an opening means through which the gene-binding agent can be inserted.
When not employing an electrochemically active agent as the gene-binding agent, there is no need to provide an electrode or terminal on cover member 5.
In gene detection tool 10 of the present invention shown in Fig. 1, electrode 51 is provided on cover member 5; it can also be provided in the reaction chamber.

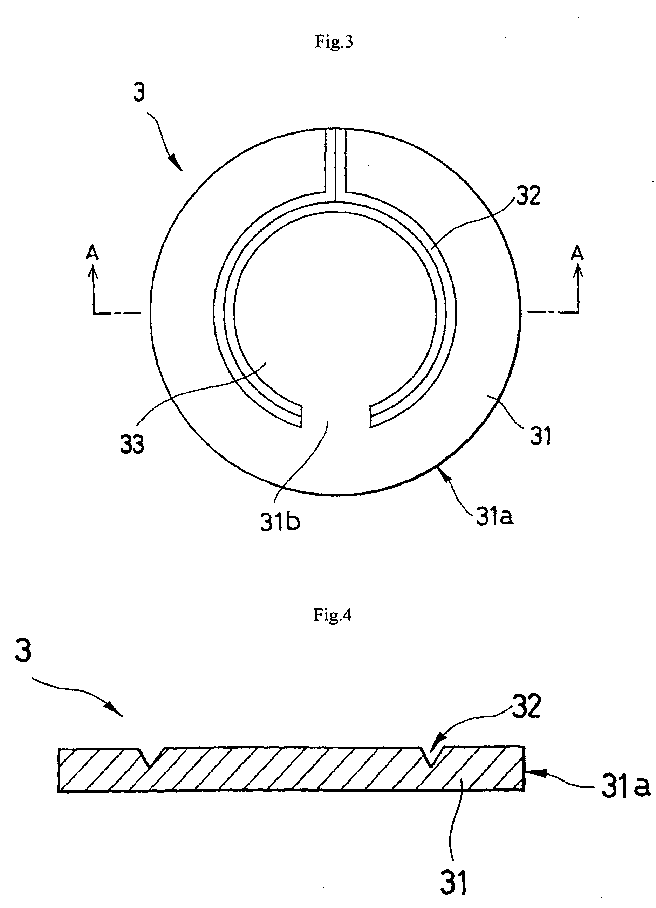

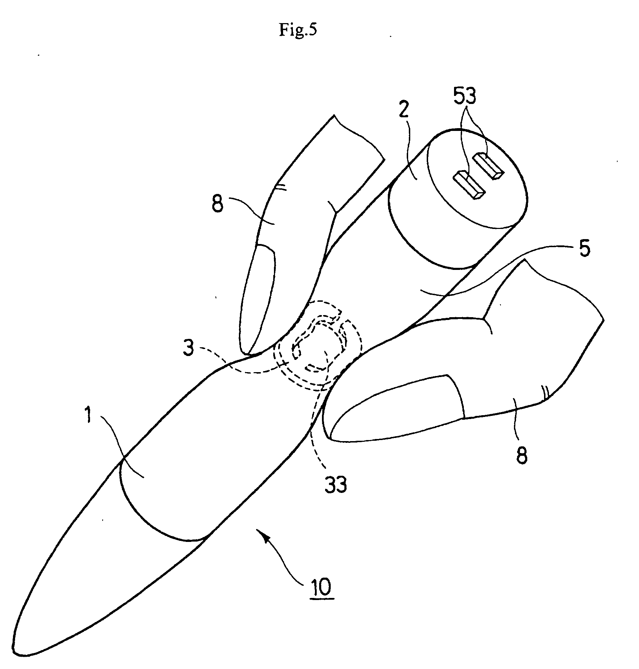

One embodiment of partition member 3 is shown in Figs. 3 to 5. Fig. 3 is a plane view of partition member 3. Fig. 4 is a sectional view along section line A-A of Fig. 3. Fig. 5 is a schematic perspective view showing partition member 3 in an unsealed state resulting from an operation of applying pressure from the exterior.

As shown in Figs. 3 and 4, partition member 3 is comprised of a thin film member 31 and a groove 32 formed on one of the surfaces thereof, meeting the inner sidewall of cylinder with sections 12 at perimeter portion 31a of thin film member 31. Groove 32 is provided to permit the ready rupturing of the seal by means of an external pressure operation (see Fig. 5), and, as shown in Fig. 4 for example, may be V-shaped. As shown in Fig. 3 for example, a groove 32 may be provided in the center 33 of thin film portion 31 so as to form a cylindrical opening. A non-rupturing portion 31b is desirably provided so that center portion 33 does not detach from thin film portion 31 when groove 32 is ruptured.

When the partition member 3 shown in Figs. 3 and 4 is provided as the partition member 3 of gene detection tool 10 of the present invention shown in Figs. 1 and 2, pressure can be applied from outside partition member 3 with a finger 8 to readily rupture thin film member 31 at the groove 32 portion, as shown in Fig. 5, thereby forming a round opening in the center. In a case where a nonrupturing portion 3 1b is provided, center portion 33 does not detach from thin film member 31, but a groove 32 is desirably formed in advance on the surface of the detection chamber 2 side of thin film member 31 so that a pressing operation from the exterior causes center portion 33 of thin film member 31 to fall to the detection chamber 2 side, as shown in Fig. 5, thus allowing solution to pass readily from reaction chamber 1 to detection chamber 2.

The operation when implementing the gene detection method of the present invention using gene detection tool 10 of the present invention shown in Figs. 1 to 5 is described below.
First, cylinder with bottom 11 is removed from cylinder with sections 12, test sample and gene amplification reaction solution 6 are inserted into cylinder with bottom 11, and cylinder with bottom 11 is fit back into cylinder with sections 12 to place reaction chamber I in a sealed state (see Figs. 1 and 2). Cover member 5 is also removed from cylinder with sections 12, solution 7 containing a gene-binding agent is inserted into the detection chamber 2 side of cylinder with sections 12, and cover member 5 is refitted onto cylinder with sections 12 to place detection chamber 2 in a sealed state (see Figs. 1 and 2).

As shown in Fig. 1, when conducting an examination with a gene detection tool 10 prepared by packing gene amplification reaction solution 6 in advance in reaction chamber 1 and packing solution 7 containing gene-binding agent in detection chamber 2, cylinder with bottom 11 can be removed from cylinder with sections 12, the test sample can be inserted into cylinder with bottom 11, and cylinder with bottom 11 can be fixed back onto cylinder with sections 12 to seal reaction chamber 1.

Next, the entire gene detection tool 10, or at least the reaction chamber 1 portion thereof, is placed in a suitable incubator (such as that employed in PCR) and the gene to be detected in the test sample is amplified. In the step of amplifying the gene to be detected in the test sample, the relative positioning of reaction chamber 1 and detection chamber 2 is not restricted. As shown in Figs. 1 and 2, reaction chamber 1 may be positioned below and detection chamber 2 above, or conversely, reaction chamber 1 may be positioned above and detection chamber 2 below. Alternatively, reaction chamber 1 and detection chamber 2 may be placed side-by-side horizontally or on a vertical incline.

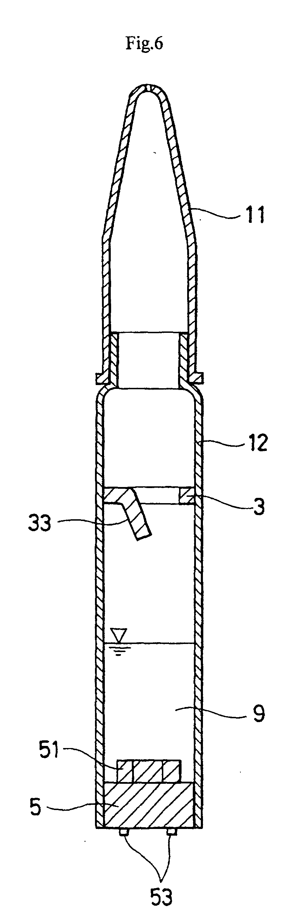

After the elapse of a period of time adequate for amplification of the gene to be detected, gene detection tool 10 is removed from the incubator and, as shown in Fig. 5, partition member 3 is unsealed by the application of pressure from the exterior. Next, the reaction solution in reaction chamber 1 is caused to move into detection chamber 2, bringing it into contact with preinserted solution 7 containing a gene-binding agent in the interior of detection chamber 2. The reaction solution can be moved from reaction chamber 1 to detection chamber 2 by, for example, pressing the reaction chamber 1 side to move the reaction solution to the detection chamber 2 side, or by holding the front end of the reaction chamber 1 side in the hand and shaking the entire gene detection tool 10 once or twice, thereby readily displacing the reaction solution. As shown in Fig. 6, a mixed solution 9 of reaction solution from reaction chamber 1 and solution 7 containing gene-binding agent in detection chamber 2 is thus formed in detection chamber 2. By bringing mixed solution 9 into contact with electrode 51, an electrochemical response can be picked up on the exterior through terminal 53.

In the gene detection tool of the present invention, the shapes of the reaction chamber and detection chamber are not restricted to the cylindrical shapes shown in Figs. 1 to 6; any shape may be employed (for example, a square columnar shape). Further, the gene detection tool of the present invention is not restricted to a monochannel form where there is only a single combination of reaction chamber, detection chamber, and partition chamber; multichannel forms comprising two or more combinations of reaction chamber, detection chamber, and partition chamber are also possible. In the case of a multichannel configuration, the control test described further below can be implemented under identical conditions.

Nor are the dimensions of the gene detection tool of the present invention limited within the range permitting the detection of genetic material in the test sample. For example, in the cylindrical gene detection tool 10 shown in Figs. 1 to 6, the total length of gene detection tool 10 (from closed end 11a (see Fig. 2) of cylinder with bottom 11 to end 5b of cover member 5 (see Fig. 2)) can be about 2 to 10 cm (more particularly, about 3 to 7 cm), the total length of reaction chamber 1 (from closed end 11a of cylinder with bottom 11 (see Fig. 2) to partition member 3) can be about 1 to 5 cm, and the total length of detection chamber 2 (from partition member 3 to end 5a of cover member 5 (see Fig. 2)) can be about 1 to 5 cm. Further, the internal diameter of cylindrical gene detection tool 10 shown in Figs. 1 to 6 can be, for example, about 3 to 10 mm. However, the gene detection tool of the present invention may also be larger or smaller than the above-stated dimensions.

Nor is the material of the gene detection tool of the present invention limited. For example, it may be molded out of a plastic material (for example, polyolefin, particularly polyethylene; polyester, particularly polyethylene terephthalate; or polyacrylate, particularly polymethyl methacrylate). Manufacturing out of a transparent material is desirable to permit observation of the reaction state in the reaction chamber and detection state in the detection chamber from the exterior.
Cylinder with sections 12 having the partition member 3 shown in Figs. 3 and 4 can be manufactured, for example, by connecting a cylinder containing partition member 3 as a closed end with a cylinder having two open ends.
Further, the material of the gene detection tool of the present invention is suitably selected from among materials having a degree of flexibility permitting the operation of pressing at least the area in the vicinity of the partition member from the perspective of applying pressure to break the seal of the partition member.

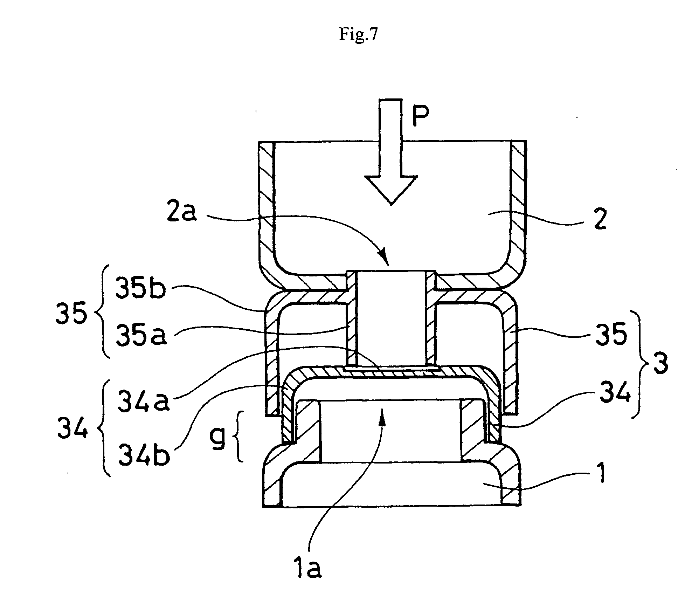

In addition to the partition member shown in Figs. 3 and 4, any means permitting the breaking of the seal by an external operation can be employed as the partition member positioned between the reaction chamber and the detection chamber in the gene detection tool of the present invention. For example, as shown in Fig. 7 (a schematic sectional view), a partition member 3 comprising a closing film support cap 34 and a protrusion support cap 35 positioned between open end 1a of reaction chamber 1 and open end 2a of detection chamber 2 may also be employed. Closing film support cap 34 comprises a closing film 34a and a closing film support frame 34b, and can be installed on the open end 1a of reaction chamber 1. Protrusion support cap 35 is comprised of a protrusion 35a and a protrusion support frame 35b, and can be installed on the open end 2a of detection chamber 2. Protrusion support frame 35 b is installed with a gap g provided between the end of protrusion support frame 35b and the perimeter portion of reaction chamber 1. As necessary, a suitable stopper means (not shown) can be provided to maintain gap g until the seal is broken.

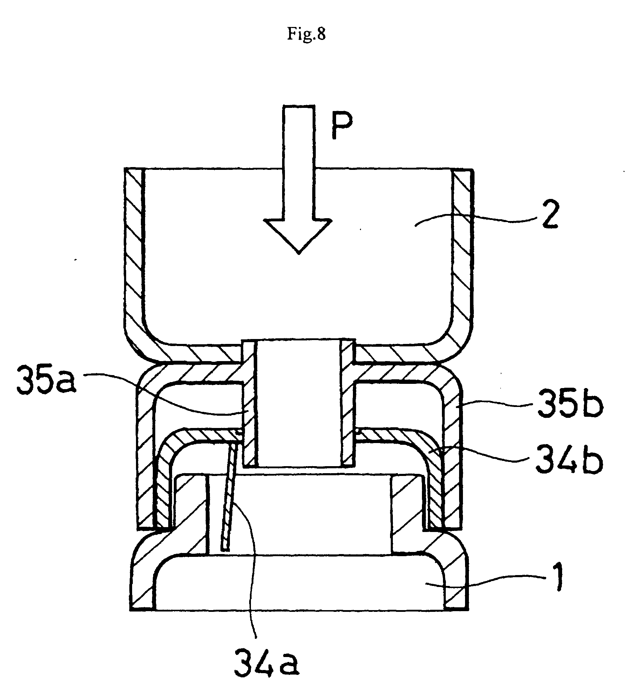

When breaking the seal of partition member 3 shown in Fig. 7, when the stopper means provided as necessary is removed and the detection chamber 2 side is pushed in the direction of the reaction chamber 1 side (the direction of arrow P), as shown in Fig. 8 (a schematic sectional view), protrusion 35a supported by protrusion support cap 35 strikes closing film 34a supported by closing film support cap 34, rupturing it.

When the partition member 3 shown in Figs. 7 and 8 is employed, removing closing film support cap 34 from the open end 1a of reaction chamber 1 can serve as the opening means for insertion of the test sample and gene amplification reaction solution. Thus, there is no need to provide fitting member 4 in the mode shown in Figs. 1 to 6. Further, removing protrusion support cap 35 from the open end 2a of detection chamber 2 can serve as the opening means for insertion of the gene-binding agent.

Genes to be detected that are suited to application of the present invention are not specifically limited so long as they are genes for which a primer for the gene amplification reaction can be designed and, using this primer, a gene amplification reaction can be conducted. Examples are naturally existing genes (such as genes derived from animals, plants, microbes, and viruses), as well as artificially manufactured genes (such as chemically amplified genes and genetically engineered genes). In the present Specification, the term "gene" is used to encompass both DNA and RNA.

Further, test samples suited to application of the present invention are not specifically limited so long as they potentially contain a gene to be detected. Examples are biological samples (such as animal (including human) body fluids (such as blood, serum, plasma, marrow fluid, tears, sweat, urine, pus, and sputum); excretions (for example, feces); organs; tissue; animals and plants themselves; and dried products of animals and plants) and samples originating from the environment (for example, river water, lake water, swamp water, seawater, and soil). Since the present invention permits detection in a sealed state, it is particularly suited to application to dangerous test samples and test samples presenting a risk (for example, test samples originating from patients infected with viruses).

The term "gene-binding agent" as employed in the present invention is not specifically limited beyond agents capable of binding to a gene and generating a detectable signal. Examples of agent suitable for use are:
(1) electrochemically active agents capable of specifically binding genes;
(2) fluorescent dyes capable of specifically binding genes; and
(3) probes having a region capable of sequence-specifically binding a gene to be detected and pairs of fluorescent groups and quenchingquenching groups, incapable of generating fluorescence prior to binding to the gene to be detected, but capable of generating fluorescence once bound to the gene to be detected.

Electrochemically active agents capable of specifically binding genes specifically bind to a gene and generate a detectable electrochemical signal. That is, they specifically bind to genes without binding to substances other than genes, and exhibit oxidizing or reducing activity in electrochemical measurement. When a specific voltage is applied to a sample solution in which are present genes to which electrochemically active agents have bound, a signal is obtained that is proportional to the quantity of gene-binding agent.

The electrochemically active agents employed in the present invention are not specifically limited so long as they are capable of specifically binding to genes and exhibit electrochemical activity. Preferred examples are electrochemically active intercalating agents capable of specifically binding to double-stranded DNA. The term "specifically binding to double-stranded DNA" means not binding to single-stranded DNA but binding to genes.

Examples of electrochemically active gene-binding agents suitable for use in the present invention are bisbenzimide derivatives, ferrocene derivatives, quinone derivatives, indophenol derivatives, acridine derivatives, flavine derivatives, viologen derivatives, ruthenium complexes, osmium complexes, cobalt complexes, platinum complexes, copper complexes, actinomycin D, domomycin, and derivatives thereof.

The fluorescent dye capable of specifically binding genes specifically binds genes (that is specifically binds genes without binding substances other than genes) and generates detectable fluorescence. Known dyes may be employed as this fluorescent dye; examples of dyes suitable for use are ethidium bromide and SYBR Green I. For example, when ethidium bromide is intercalated into nucleic acid (either single-stranded or double-stranded), it generates 590 nm fluorescence when excited by 260 nm light, and this fluorescence can be used for detection. Similarly, SYBR Green I specifically binds DNA, generating fluorescence at a wavelength of 519 nm when excited at a wavelength of 494 nm.

The probe having a region capable of sequence-specifically binding a gene to be detected and pairs of fluorescent groups and quenching groups, incapable of generating fluorescence prior to binding the gene to be detected, but capable of generating fluorescence once bound to the gene to be detected specifically binds a particular DNA sequence in the gene to be detected (that is, hybridizes with a particular DNA sequence in the gene to be detected) and generates detectable fluorescence. An example of such a probe that is suitable for use is the probe employed in the molecular beacon method. Fluorescent groups and quenching groups are present within the probe molecule. A DNA sequence region capable of sequence-specifically binding the gene to be detected and a DNA sequence region capable of self-hybridization are also present within this probe. When the probe is in an intramolecular self-hybridized state, the fluorescent groups and quenching groups are in close proximity to each other, precluding the generation of fluorescence even by supplying an excitation light beam. However, when the probe hybridizes in a sequence-specific manner with the gene being detected, the spacing between the fluorescent groups and the quenching groups widens, permitting the generation of fluorescence when excitation light is supplied to the fluorescent group.

When an electrochemically active agent is employed as the gene-binding agent in the present invention, an electrode must be provided in the detection chamber. However, when employing a fluorescent dye or a probe capable of generating fluorescence, no electrode need be provided in the detection chamber. A state permitting measurement by a fluorophotometer is required; for example, a material capable of passing excitation light and fluorescence must be used in the configuration, and a shape that can be loaded into a fluorophotometer measurement element must be employed.

In the present invention, a gene amplification reaction is conducted in the reaction chamber using:
(a) a test sample potentially containing the gene to be detected; and
(b) a gene amplification reaction solution comprising at least a primer capable of amplifying the gene using the gene to be detected as template. The gene amplification reaction conducted in the present invention is not specifically limited so long as it permits the amplification of a gene binding with the gene-binding agent. Examples are gene amplification reactions, replication reactions, transcription reactions, and reverse transcription reactions. Gene amplification reactions are preferred.

Any known gene amplification method may be employed. Examples are polymerase chain reaction (PCR), loop-mediated isothermal amplification (LAMP), isothermal and chimeric primer-initiated amplification of nucleic acids (ICAN), transcription-mediated amplification (TMA), strand displacement amplification (SDA), ligase chain reaction (LCR), and nucleic acid sequence-based amplification (NASBA).

Generally, in gene amplification reactions, the gene amplification reaction is blocked when a gene-binding agent is present. That is, when either the above-described electrochemically active agent or fluorescent dye is present during a gene amplification reaction, the gene amplification reaction is blocked. However, since the gene amplification reaction is conducted in the absence of the gene-binding agent in the present invention, even when few copies of the gene to be detected are present in the test sample, the genetic material can be detected with high sensitivity.

In the present invention, the gene amplification reaction itself can be conducted in precisely the same manner as a common gene amplification reaction. For example, in the present invention, when conducting PCR as the gene amplification reaction, it can be conducted in the usual manner.

When the genetic material to be detected is DNA, for example, PCR can be conducted using heat-resistant DNA polymerase (for example, Taq polymerase), conducting an initial denaturation reaction (for example, 2 to 3 minutes at 97°C), and then (1) conducting a DNA denaturation step (30 seconds at 90 to 94°C), (2) conducting a step of annealing the single-stranded DNA and primer (30 seconds at 50 to 55°C), (3) conducting a DNA amplification step with heat-resistant DNA polymerase (1 to 2 minutes at 70 to 75°C), and then repeating this amplification cycle (for example, 15 to 45 times).

When the genetic material to be detected is RNA, for example, the reverse transcriptase PCR (RT-PCR) method can be implemented. That is, using reverse transcriptase and oligo (dT) primer, a reverse transcription reaction is conducted, after which, in the same manner as for DNA, heat-resistant DNA polymerase (such as Taq polymerase) is employed to conduct an initial denaturation reaction, and an amplification cycle is conducted repeatedly.

In the present invention, the solution produced by the gene amplification reaction from the test sample and the solution containing the gene-binding agent are mixed in the detection chamber, and the electrochemical response, fluorescence, or the like of this mixed solution is measured. For example, when PCR is conducted as the gene amplification reaction, the solution produced by polymerase chain reaction from the test sample and the solution containing the gene-binding agent (for example, an intercalating agent) are mixed in the detection chamber and the electrochemical response, fluorescence, or the like of this mixed solution is measured.

In addition, a control test, in which the same operations are repeated with the exception that the gene amplification reaction is not conducted, is desirably conducted in the present invention. That is, the test sample is contacted with the gene amplification reaction solution in the reaction chamber, but under conditions not permitting amplification of the gene to be detected; the partition member is unsealed; the mixed solution of the test sample and the gene amplification reaction solution is moved into the detection chamber, where it is mixed with the solution containing the gene-binding agent; and the electrochemical response or fluorescence of the mixed solution is measured.

In the present invention, measurement of the electrochemical response or the level of fluorescence can be conducted by measuring peak currents when a voltage is applied to, and measuring the level of fluorescence generated when an excitation light beam is directed onto, the mixed solution (for example, a mixed solution of the solution generated by the gene amplification reaction and the solution containing the gene-binding agent) in the detection chamber.
Examples of specific methods of measuring electrochemical response are linear sweep voltammetry (LSV), Chrono amperometry (CA), Chrono coulometry (CC), and cyclic voltammetry (CV).

In detection employing an electrochemical response in the present invention, the absence or presence of the gene to be detected in the test sample can be determined based on the following principles.
That is, the gene-binding agent that is packed into the detection chamber is capable of specifically binding genetic material and exhibits electrochemical activity. Accordingly, when the reaction solution sent from the reaction chamber does not contain the gene or contains only a small quantity of the gene, the electrochemical response in the mixed solution generated in the detection chamber can be compared to the electrochemical response in the mixed solution generated in the detection chamber when a large quantity of the gene is contained in the reaction solution sent from the reaction chamber. Since the gene-binding solution binds the gene in the latter case, the electrochemical response of the latter is lower than the electrochemical response of the former, and an electrochemically detectable difference is produced.

Accordingly, when the gene is amplified by gene amplification reaction in the reaction chamber and the gene is present in large quantity in the reaction solution, the electrochemical response in the mixed solution generated in the detection chamber is lower than the electrochemical response of the mixed solution generated in the detection chamber in the control test (that is, the test implemented without conducting a gene amplification reaction in the reaction chamber). In that case, the gene to be detected can be determined to be present in the test sample.

When a large quantity of gene is not present in the reaction solution and the gene is not amplified by gene amplification reaction in the reaction chamber, no difference is produced between the electrochemical response in the mixed solution generated in the detection chamber and the electrochemical response in the mixed solution in the control test. In that case, the gene to be detected can be determined not to be present in the test sample.

For example, when conducting PCR as the gene amplification reaction, an intercalating agent capable of specifically binding double-stranded DNA by intercalating between a pair of adjacent bases in double-stranded DNA can be employed as the gene-binding agent.

When DNA is being amplified by PCR and double-stranded DNA is present in large quantity in the reaction solution, the electrochemical response in the mixed solution in the detection chamber will be lower than the electrochemical response in the mixed solution of the control test. In that case, the gene to be detected can be determined to be present in the test sample.

Further, when not amplifying DNA by PCR and a large quantity of double-stranded DNA is not present in the reaction solution, no difference is produced between the electrochemical response in the mixed solution in the detection chamber and the electrochemical response in the mixed solution in the control test. In that case, the gene to be detected can be determined to not be present in the test sample.
In detection with the present invention using a fluorescent dye, as well, it is possible to determine whether a gene being detected is present or not in a test sample based on the same principle as in detection by electrochemical response.

That is, the gene-binding agent that is packed into the detection chamber specifically binds to the gene. When it binds the gene, the generation of fluorescence becomes possible. When it does not bind the gene, the generation of fluorescence is impossible. Accordingly, when the reaction solution sent from the reaction chamber does not contain the gene, fluorescence is not generated even when the mixed solution generated in the detection chamber is irradiated with excitation light. Further, when there is only a small quantity of gene contained in the reaction solution sent from the reaction chamber, only a small amount of fluorescence is generated when irradiated with excitation light.

By contrast, when a large quantity of gene is contained in the reaction solution sent from the reaction chamber and the reaction solution sent from the reaction chamber is irradiated with excitation light, a large amount of fluorescence is generated based on the quantity of gene. This difference in fluorescence can be compared to determine whether or not the gene to be detected is present in the test sample.

In detection with the present invention using probe capable of generating fluorescence, it is possible to determine whether or not the gene to be detected is present in the test sample based on the same principle as in detection using the above fluorescent dye.

That is, the gene-binding agent that is packed in the detection chamber is capable of binding in a sequence-specific manner the gene to be detected. When hybridized with the gene to be detected, it is capable of generating fluorescence, and when not hybridized with the gene to be detected, it is not capable of generating fluorescence. Accordingly, when the reaction solution sent from the reaction chamber does not contain the gene to be detected, no fluorescence is generated due to the effect of the quenching group even when the mixed solution generated in the detection chamber is irradiated with excitation light. Further, when the quantity of gene to be detected that is contained in the reaction solution sent from the reaction chamber is low, only a small amount of fluorescence is generated even when irradiated with excitation light.

By contrast, when a large quantity of the gene to be detected is contained in the reaction solution sent from the reaction chamber and the mixed solution generated in the reaction chamber is irradiated with excitation light, a large amount of fluorescence is generated based on the quantity of the gene to be detected. By comparing this difference in fluorescence, it is possible to determine whether or not the gene to be determined is present in the test sample.

Further, when conducting detection using a probe capable of generating fluorescence, since the probe employed in the reaction chamber specifically amplifies the gene to be detected and since this probe recognizes in a sequence specific manner the gene to be detected even in the detection chamber, more specific detection is possible and the precision of detection is enhanced.

The gene detection kit of the present invention contains a gene amplification reaction solution or gene amplification reagent and a gene-binding agent binding the gene and generating a detectable signal in addition to the above-described gene detection tool of the present invention. The gene amplification reaction solution, gene amplification reagent, and the gene-binding agent that binds a gene and generates a detectable signal are identical to those set forth above. The gene amplification reaction solution, gene amplification reagent, and the gene-binding agent that binds a gene and generates a detectable signal may be packed in advance into specific chambers in the gene detection tool. Alternatively, they may be placed in separate containers from the gene detection tool and inserted into prescribed chambers of the gene detection tool from the individual containers during use.

The gene detection kit of the present invention may also contain a means of gene purification. Known means of gene purification may be employed. Examples are spin columns with membrane filters, purification columns, cleaning solutions, and dissolving solutions. However, this is not a limitation.

### Embodiments

The present invention is specifically described below through embodiments; however, the scope of the present invention is not limited thereby. The parts in the blending quantities given in the embodiments denote weight.

### Embodiment 1

### (Detection of hepatitis B virus DNA)

In the present embodiment, a gene detection tool (total length of gene detection tool = about 5 cm; total length of reaction chamber = about 2.5 m; total length of detection chamber = about 1.5 cm; internal diameter of reaction chamber and detection chamber = about 6 mm) identical to gene detection tool 10 of the present invention shown in Figs. 1 to 6 was employed. The gene was amplified by the LAMP method and detected by an electrochemical method.

A suspension containing a plasmid into which the entire genome sequence of the hepatitis B virus ("HBV" hereinafter) had been inserted was employed as the test sample. A 202 bp section of the HBV (7575 bp) DNA sequence, from number 178 to number 380, was employed as the target DNA using the LAMP method.

Initially, cover member 5 was removed from cylinder with sections 12, a 240 microliter quantity of aqueous solution ("Hoechst 33258 aqueous solution" hereinafter) containing 60 micromol/L of a compound (Hoechst 33258; Hoechst Corp.) denoted by: 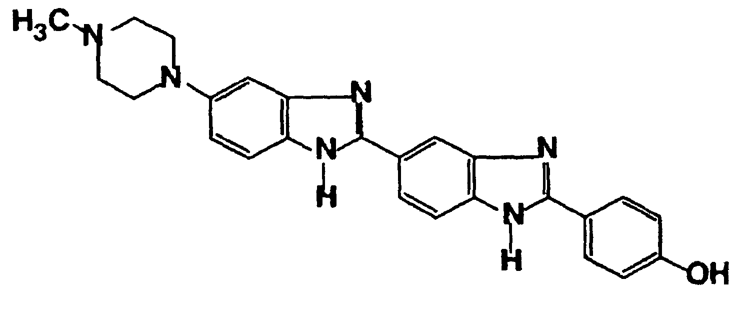 was inserted into the detection chamber 2 side of cylinder with sections 12, cover member 5 was refitted onto cylinder with sections 12, and detection chamber 2 was sealed.

Next, cylinder with bottom 11 was removed from cylinder with sections 12, a 23 microliter quantity of LAMP solution (40 units of large Bst polymerase fragments (made by New England BioLabs Corp.), 5 pmol of primer B3, 5 pmol of primer F3, 20 pmol of FIP, 20 pmol of BIP, and buffer), and 2 microliters of test sample (equivalent to 10 pg of HBV plasmid) were introduced, cylinder with bottom 11 was refitted onto cylinder with sections 12, and reaction chamber 1 was sealed.

Gene detection tool 10 was installed on a PCR thermal cycler (Takara Shuzo) and LAMP was conducted. The LAMP reaction was conducted by reacting at 65°C for 1 hour followed by reacting for 10 minutes at 80°C.

Following completion of the LAMP reaction, as shown in Fig. 5, the seal of partition member 3 was broken by an operation of pressing from the exterior with fingers, the front end on the reaction chamber I side was held manually, and the entire gene detection tool 10 was shaken once or twice to move the reaction solution in reaction chamber 1 into detection chamber 2, where it came into contact with solution 7 containing a gene-binding agent that had been preinserted into detection chamber 2. With the mixed solution 9 obtained in a state of contact with an electrode 51, the tool was mounted on a potentiostat and the electrochemical response was picked up on the exterior via terminal 53.

The electrochemical response in detection chamber 2 was measured by linear sweep voltammetry (LSV). In LSV measurement, the sweeping rate was 100 mV/s from 200 to 900 mV, and the current value was measured. The area of the electrode was 0.8 mm².

As a control test, the above operation was repeated with the exception that the seal of partition member 3 was broken without conducting a LAMP reaction.

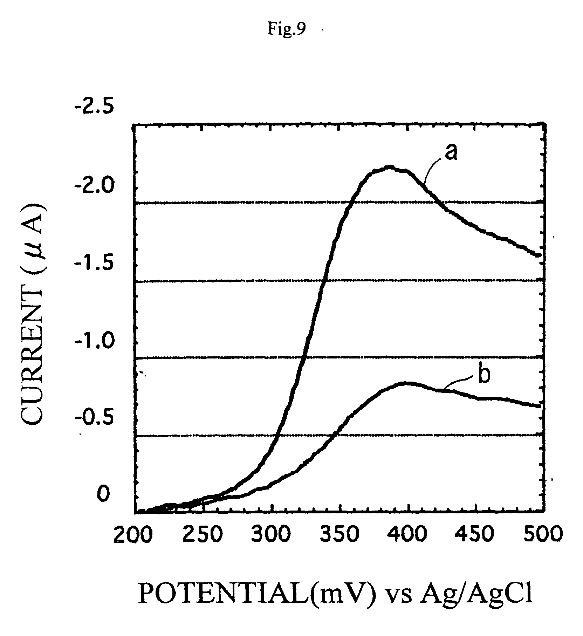

The results of LSV measurement are given in Fig. 9. In Fig. 9, curve *a* is the result when no LAMP reaction was conducted (control), and curve b is the result when a LAMP reaction was conducted.

In LSV measurement, the oxidizing current of the compound (Hoechst 33258) denoted by the above formula was measured. The oxidizing current value was dependent on the concentration of Hoechst 33258. Further, Hoechst 33258 is known to form complexes with DNA, and the complexes with DNA tend to aggregate. Accordingly, when the LAMP reaction amplifies DNA, this compound combines with DNA, and the amount of aggregated Hoechst 33258 increases. As a result, the greater the amount of complex with DNA becomes, the lower the apparent concentration of Hoechst 33258 becomes, and the lower the oxidizing current value in LSV measurement. Reduction in the oxidizing current value accompanying the addition of DNA occurs linearly in response to the amount of DNA added. For example, it is possible to quantify DNA over a detection range of 1.5 to 25 ng/mL at a resolution of 1.5 ng/mL.

In the results shown in Fig. 9, curve b, showing the results obtained when a LAMP reaction was conducted, exhibits a lower oxidizing current value than curve *a*, showing the results when no LAMP reaction was conducted (control test). The LAMP reaction amplified the HBV genome, which was the target DNA, and the Hoechst 33258 aggregated. As a result, the oxidizing current value was thought to have dropped.

### Industrial Applicability

Since the present invention permits the handling of test samples in a closed system, the risk of infection to the processor is reduced and waste products generated by examination are readily processed, even when handling dangerous samples at medical treatment facilities and clinical examination facilities. Further, since the gene amplification reaction is conducted in the absence of a gene-binding agent such as an intercalating agent, the gene amplification reaction is not blocked. This is advantageous from the perspective of permitting the highly sensitive detection of even trace quantities of genetic material. It is further possible to detect genes having a specific DNA sequence in trace quantity samples (for example, less than or equal to 2 microliters or less than or equal to 1 microliter), permitting the configuration of a simpler, more compact device.

## Claims

1. A gene detection tool comprising a sealable reaction chamber and a sealable detection chamber and a partition member positioned between the reaction chamber and the detection chamber that separates the reaction chamber and the detection chamber, wherein
the partition member is capable of unsealing;
the reaction chamber is comprised of at least two members capable of forming a sealed reaction chamber by mutually fitting an opening means provided therein; and
the detection chamber or reaction chamber comprises a gene detection element.

2. The gene detection tool according to claim 1, wherein the partition member comprises a groove-shaped notch in a portion of the surface thereof; and at least the reaction chamber and the detection chamber adjacent to the partition member are formed of a flexible material, thereby the operation of applying pressure to the tool from the exterior ruptures at least a portion of the notch, unsealing the partition member.

3. The gene detection tool according to claim 1 or 2, wherein the reaction chamber is comprised of a cylindrical member one end of which communicates with the detection chamber and is sealed off by the partition member and the other end of which is comprised of an opening means, and a cylindrical member having a bottom, one end of which is closed and the other end of which is an opening means; and these two opening means fit together to seal off the reaction chamber.

4. The gene detection tool according to claim 3, wherein the reaction chamber has a gene detection member on the end that is closed off by the cylindrical member having a bottom.

5. The gene detection tool according to any one of claims 1 to 3, wherein the detection chamber is comprised of a cylindrical member, one end of which communicates with the reaction chamber and is sealed off by the partition member and the other end of which is comprised of an opening means; and a tightly fitting cover comprising a gene detection member is provided in the opening means.

6. The gene detection tool according to any one of claims 1 to 5, wherein the reaction chamber is one in which test sample and gene amplification reaction solution is packed.

7. The gene detection tool according to any one of claims 1 to 6, wherein the detection chamber is one in which a gene-binding agent that binds genes and generates a detectable signal is packed.

8. The gene detection tool according to claim 7, wherein unsealing of the partition member creates a passage for mixing the test sample and gene amplification reaction solution in the reaction chamber with the gene-binding agent that binds genes and generates a detectable signal in the detection chamber.

9. The gene detection tool according to any one of claims 1 to 8, wherein the gene detection member is an electrochemical detection member or a fluorescence-detecting member.

10. A gene detection tool comprising a mutually independent sealable reaction chamber and detection chamber, and a partition member positioned between the reaction chamber and the detection chamber that seals off the reaction chamber and detection chamber from each other, and is **characterized in that**:
(a) the reaction chamber comprises an opening means permitting the insertion of a test sample potentially containing a gene to be detected and a gene amplification reaction solution;
(b) the detection chamber comprises an opening means permitting the insertion of a gene-binding agent that binds a gene and generates a detectable signal;
(c) the partition member is capable of unsealing by an operation from the exterior while maintaining the overall sealed state of the gene detection tool comprising the reaction chamber and detection chamber; and
(d) when the partition member is unsealed, the passage generated by this unsealing allows the solution in the reaction chamber to move into the detection chamber.

11. The gene detection tool according to any one of claims 7 to 10, wherein the gene-binding agent is selected from among the group consisting of:
(a) electrochemically active agents capable of specifically binding genes;
(b) fluorescent dyes capable of specifically binding genes; and
(c) probes having a region capable of sequence-specifically binding a gene to be detected and pairs of fluorescent groups and quenching groups, incapable of generating fluorescence prior to binding the gene to be detected but capable of generating fluorescence once bound to the gene to be detected.

12. A gene detection method in which a gene amplification operation and a gene detection operation are conducted in a sealed state using a gene detection device comprising a sealable reaction chamber and a sealable detection chamber, having a partition member which is positioned between the reaction chamber and the detection chamber and which separates the reaction chamber from the detection chamber, with test sample and gene amplification reaction solution being packed in said reaction chamber and a gene-binding agent capable of binding with a gene and generating a detectable signal being packed in said detection chamber, wherein:
(1) at least the reaction chamber is imparted with conditions capable of amplifying the gene to be detected;
(2) following the operation of (1), the partition member is unsealed while maintaining the gene detection tool in a sealed state;
(3) through a passage created in the partition member by the unsealing, the solution in the reaction chamber is brought into contact with the gene-binding agent capable of binding the gene in the detection chamber and generating a detectable signal; and
(4) the signal obtained is detected.

13. The gene detection method according to claim 12, wherein the gene-binding agent is selected from among the group consisting of:
(a) electrochemically active agents capable of specifically binding genes;
(b) fluorescent dyes capable of specifically binding genes; and
(c) probes having a region capable of sequence-specifically binding a gene to be detected and pairs of fluorescent groups and quenching groups, incapable of generating fluorescence prior to binding the gene to be detected but capable of generating fluorescence once bound to the gene to be detected.

14. The gene detection method according to claim 12, wherein the detection of signal is electrochemical detection or fluorescent detection.

15. The gene detection method according to any one of claims 12 to 14, wherein the gene amplification reaction solution is prepacked into the reaction chamber and the test sample is inserted into the reaction chamber prior to the operation of (1).

16. The gene detection method according to any one of claims 12 to 15, wherein the reaction chamber has an opening means, the test sample and/or gene amplification reaction solution is inserted into the reaction chamber through the opening means, and following insertion, the opening means is closed to seal the reaction chamber.

17. The gene detection method according to any one of claims 12 to 16, wherein the gene-binding agent that binds a gene and generates a detectable signal is packed in the detection chamber.

18. A gene detection method **characterized by** comprising the steps of:
(1) inserting a test sample potentially containing the gene to be detected and the gene amplification reaction solution through an opening means into the reaction chamber and then closing the opening means to seal the reaction chamber;
(2) imparting conditions permitting amplification of the gene to be detected to the interior of the reaction chamber;
(3) following the elapse of a period of time adequate for amplification of the gene to be detected and while maintaining the gene detection tool comprising the reaction chamber and detection chamber in an overall sealed state, unsealing by an external operation the partition member that is positioned between the reaction chamber and the detection chamber and seals off the reaction chamber from the detection chamber;
(4) causing the reaction solution in the reaction chamber to move into the detection chamber through a passage created by the unsealing;
(5) contacting the gene-binding agent that binds a gene and generates a detectable signal with the reaction solution in the detection chamber; and
(6) detecting the signal obtained.

19. A gene detection kit comprising the gene detection tool according to any one of claims 1 to 11, a gene amplification reaction solution or gene amplification reagent, and a gene-binding agent capable of binding a gene and generating a detectable signal.

20. The gene detection kit according to claim 19, wherein the kit further comprises a means of purifying genes.
